# EUROPEAN PATENT APPLICATION

(11) **EP 4 825 497 A1**
(43) Date of publication of application: **21.10.2026**
(21) Application number: 26171332.5
(22) Date of filing: 09.04.2026
(51) Int. Cl.: B04B 13/00, A61M 1/36

(54) **A BIOLOGICAL FLUID PROCESSING DEVICE AND METHOD FOR DETECTING THE PROPER LOADING OF A CENTRIFUGE SEPARATION CHAMBER**

(30) Priority: 10.04.2025 US 202563786690 P
(71) Applicant: Fenwal, Inc., Lake Zurich, IL 60047 (US)
(72) Inventor: QUEZADA, Francesca S., Lake Zurich, 60047 (US); MAHER, Jeffrey R., Lake Zurich, 60047 (US); DICOLA, Nicholas R., Lake Zurich, 60047 (US)
(74) Representative: Maikowski & Ninnemann Patentanwälte Partnerschaft mbB

(57) **Abstract**

Systems and methods are provided for detecting the proper loading of a centrifuge separation chamber, where testing at various points during spin up could reveal evidence of improper loading prior to reaching speeds of rotation where damage may be possible and/or more likely, thereby allowing for the potential for avoiding more severe consequences should the device be operated in instances where the centrifuge separation chamber has been improperly loaded. Such testing could be managed by a controller, which could take into account one or more of speed, acceleration and operational data during spin up to recognize the evidence of a potential improper load.

## Description

### Field of the Application

The present subject matter relates to systems and methods for processing biological fluids. More particularly, the present subject matter relates to detecting the proper loading of a centrifuge separation chamber within a centrifuge compartment of a centrifugal separator.

### Background

As part of processing biological fluids, such as blood, whole blood is routinely separated into various components, such as red blood cells, platelets and plasma. At least one common approach for separating blood into its various components makes use of a centrifuge and/or centrifugal separator for spinning the blood, where the varying densities of the blood components will cause the blood components to migrate into separate strata under the influence of an applied centrifugal force.

At least some biological fluid processing devices incorporate a more durable reusable portion, and a disposable portion, often limited to a single use, which includes the portions that generally come into more direct contact with the biological fluids. As part of the operation of the processing device, the operator typically loads the disposable portion on or into the more durable reusable portion prior to processing the biological fluids, and removes and discards the disposable portion after the biological fluids have been processed. This can extend to biological fluid processing devices, which incorporate centrifuges. In such an instance, the operation of the centrifuge can include a more durable reusable portion, and a disposable portion where the disposable portion of the centrifuge process can be received within the more durable reusable portion. The disposable portion can often be referred to as a centrifugal separation chamber, and the more durable reusable portion can often be referred to as the centrifuge compartment and/or bucket. Sometimes the proper operation of a biological fluid processing device can be dependent upon a correct loading of the disposable portion into the more durable reusable portion. In some instances, any attempted operation of the biological fluid processing device after an incorrect loading can result in poor performance, an operational failure, as well as in some instances, damage to the disposable and/or more durable reusable portions. Any such failure can often require corrective action, such as the reloading of the improperly loaded disposable portion, which can sometimes include the replacement of any failed or spoiled components.

Accordingly, it would be desirable to provide a system where the operation of the device can be monitored to look for symptoms of improper loading, where symptoms of improper loading could possibly be recognized reacted to and corrected prior to the operation of the biological fluid processing device having proceeded to the point where a more serious error including potential damage to the device can or has occurred.

### Summary

There are several aspects of the present subject matter which may be embodied separately or together in the devices and systems described and claimed below. These aspects may be employed alone or in combination with other aspects of the subject matter described herein, and the description of these aspects together is not intended to preclude the use of these aspects separately or the claiming of such aspects separately as set forth in the claims appended hereto.

In one aspect, a biological processing device is provided. The biological processing device includes a centrifugal separator having a centrifuge compartment adapted for receiving a centrifugal separation chamber, wherein the centrifugal separator is further adapted for spinning the received centrifugal separation chamber. The biological processing device further includes a controller, which is configured for detecting whether any centrifugal separation chamber that has been received within the centrifuge compartment of the centrifugal separator, has been correctly loaded, and generating an alert if the centrifugal separation chamber has not been correctly loaded.

In another aspect, a further biological processing device is provided. The biological processing device includes a disposable flow circuit including a centrifugal separation chamber, and a centrifugal separator having a centrifuge compartment for receiving the centrifugal separation chamber of the disposable flow circuit, wherein the centrifugal separator is adapted for spinning the received centrifugal separation chamber. The biological processing device further includes a controller configured for detecting whether any centrifugal separation chamber, that has been received within the centrifuge compartment of the centrifugal separator, has been correctly loaded, and generating an alert if the centrifugal separation chamber has not been correctly loaded.

In another aspect, a method for detecting proper loading of a centrifuge separation chamber in a biological fluid processing device is provided. The method includes receiving the centrifugal separation chamber in a centrifuge compartment of the biological fluid processing device and spinning the received centrifugal separation chamber. A controller detects whether the centrifugal separation chamber, that has been received within the centrifuge compartment of the centrifugal separator, has been correctly loaded, and if the centrifugal separation chamber has not been loaded correctly, an alert is generated.

These and other features, and advantages of the present disclosure are evident from the following description of one or more exemplary embodiments, with reference to the accompanying drawings.

### Brief Description of the Drawings

Fig. 1 is a partial block diagram of a biological fluid processing device for use in detecting the proper loading of a centrifugal separation chamber, in accordance with at least one aspect of the present application;
Fig. 2 is a partial block diagram of a biological fluid processing device for use in detecting the proper loading of a centrifugal separation chamber, in accordance with at least a further aspect of the present application;
Fig. 3 is a partial block diagram of a biological fluid processing device for use in detecting the proper loading of a centrifugal separation chamber, in accordance with at least a still further aspect of the present application;
Fig. 4 is a partial block diagram of a biological fluid processing device for use in detecting the proper loading of a centrifugal separation chamber, which incorporates various features from each of the aspects illustrated in Figs. 1, 2 and 3;
Fig. 5 is a perspective view of an exemplary blood separation device within which the detection of proper loading of a centrifugal separation chamber can be implemented;
Fig. 6 is a partial perspective view of an exemplary centrifugal separator, which can be used as part of the exemplary blood processing device illustrated in Fig. 5;
Fig. 7 is a partial perspective view of an exemplary centrifugal separator, with a light source and a light detector arranged and oriented to allow for selective light communication between the two via a light guide associated with the centrifugal separation chamber dependent upon the current relative rotation of the centrifugal separation chamber;
Fig. 8 is a perspective view of a light guide, which could be used in combination with a centrifugal separation chamber for selectively conveying light between a light source and a light detector;
Fig. 9 is a partial plan view of a cassette, which can form a part of a fluid flow circuit for use in biological fluid processing device, such as the blood separation device illustrated in Fig. 5;
Fig. 10 is a flow diagram of a method for detecting the proper loading of a centrifugal separation chamber in a centrifugal separator;
Fig. 11 is a flow diagram of a method for selecting one of one or more sets of threshold values in connection with detecting the proper loading of a centrifugal separation chamber; and
Fig. 12 is a flow diagram of a method for adjusting the value of one or more thresholds for use in connection with detecting the proper loading of a centrifugal separation chamber.

### Description of the Illustrated Embodiments

The embodiments disclosed herein are for the purpose of providing an exemplary description of the present subject matter. They are, however, only exemplary, and the present subject matter may be embodied in various forms. Therefore, specific details disclosed herein are not to be interpreted as limiting the subject matter as defined in the accompanying claims.

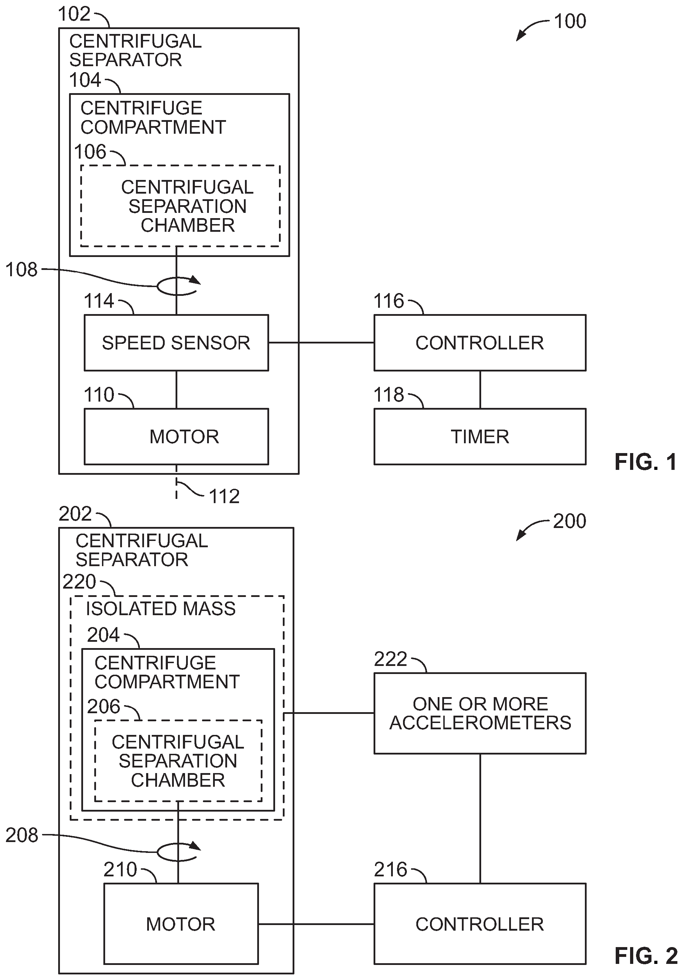

Fig. 1 illustrates a partial block diagram 100 of a biological fluid processing device for use in detecting the proper loading of a centrifugal separation chamber 106, in accordance with at least one aspect of the present application. In the illustrated embodiment, the biological fluid processing device includes a centrifugal separator 102, which has a centrifuge compartment 104, which is adapted for receiving a centrifugal separation chamber 106, and spinning 108 the same. The centrifugal separation chamber 106 is typically part of a disposable portion, sometimes referred to as a disposable flow circuit, that comes into more direct contact with the biological fluids that are being processed by the device, which can correspondingly be swapped out and/or changed to help minimize and/or avoid cross contamination relative to multiple different fluid processing jobs. However, less than proper loading of centrifugal separation chamber 106 within the centrifuge compartment 104 of the centrifugal separator 102 can lead to a less than optimal level of performance. The degree to which the centrifugal separation chamber 106 is misloaded can impact the degree to which operation of the centrifugal separator 102 is affected. For example, misloading of the centrifugal separation chamber 106 can result in wobble or orbiting, which could lead to a less efficient separation caused by changes to the separation dynamics, where when the misloading is more severe, damage to the centrifugal separator 102, the centrifugal separation chamber 106, and/or other related elements can occur. For example, an umbilicus, which is part of the fluid interface of the centrifugal separation chamber 106, could be damaged and/or broken, in some instances while attempting to operate the centrifugal separator 102 with an improperly loaded centrifugal separation chamber 106. Such a breakage could result in fluid leakage, which could involve the leakage of a potential additive solution like saline, or could result in a leakage of the particular biological fluid being processed. Any such breakage could result in a need to terminate the procedure and replace the disposable flow circuit, and could affect the particular fluid processing, which was taking place at the time.

A motor 110 which is part of the centrifugal separator 102 can be used to facilitate the intended spinning 108 of the centrifugal separation chamber 106 within the centrifuge compartment 104. Any resulting wobble and/or orbiting during a spinning 108 of the centrifugal separation chamber 106 by the motor 110 could be caused by an imbalance in the mass of the centrifugal separation chamber relative to an intended axis of rotation 112 of the motor 110, which might be present or made worse in instances where the centrifugal separation chamber has not been properly loaded. In the particular embodiment illustrated, the centrifugal separator 102 further includes a speed sensor 114, which could be used to monitor the current speed at which the centrifugal separation chamber 106 is being rotated or being spun by the motor 110. The rate of rotation during spin-up is monitored by a controller 116 with the aid of an associated timer 118 to detect any deviation from the expected performance or where the deviation might fall short by exceeding a predetermined threshold associated with a degree of acceptable variance.

For example, it may take an improperly loaded centrifugal separation chamber 106 a longer than expected amount of time to reach a desired rotation speed, such as from rest, as compared to a correctly loaded chamber 106. This may be due to an inability of the motor 110 to generate a sufficient increased level of torque, for spinning up an incorrectly loaded chamber 106 to overcome any changes to the inertia of the mass of the incorrectly loaded chamber to be rotated, relative to the intended axis of rotation 112. By observing the rate of change of the rotation speed from rest, it may be possible for the controller 116 to detect a variance in the spin-up time resulting from an improper loading of the chamber, prior to the chamber getting to full speed, where there is more of a risk of failure and/or damage to the centrifugal separator 102, the centrifugal separation chamber 106, or any the components related to the operation of the centrifugal separator. In some cases, it may take longer than normally expected to reach a particular speed. In the same or other cases, such as in the case of a jam, a particular desired speed may not be possible, and correspondingly it may be desirable to determine an improper loading after a particular time has passed and it has been determined that there has been a failure to reach a desired and/or expected speed.

For example, in some embodiments, failure to reach 100 rotations per minute within 2 seconds after a spin up of the centrifugal separation chamber 106 may be symptomatic of a misloaded chamber. Furthermore, a failure to reach 500 rotations per minute after 15 seconds could also be symptomatic of a potential problem. The particular threshold values of interest are likely dependent on the specifics related to a particular use case, such as strength of the motor, the mass of the object being rotated, as well as potentially other factors, which might impact the ability of the motor to spin up an associated mass.

It is further possible that instead of the centrifugal separation chamber 106 having been misloaded, that the chamber has not been loaded at all. In such an instance, the centrifuge may be detected as having reached a particular speed much quicker than expected, as the expected inertia of a centrifugal separation chamber 106, that is absent, having not been loaded, would be much less. Such a test could be combined with a further check that the centrifuge compartment 104 has been accessed, such as by confirming that any door providing access to the centrifuge compartment 104 for loading the centrifugal separation chamber 106 into the centrifuge compartment 104 has been both opened and then closed.

Fig. 2 illustrates a partial block diagram 200 of a biological fluid processing device for use in detecting the proper loading of a centrifugal separation chamber, in accordance with at least a further aspect of the present application. In the illustrated embodiment, the biological fluid processing device includes a centrifugal separator 202, which has a centrifuge compartment 204, which is adapted for receiving a centrifugal separation chamber 206, and spinning 208 the same by a motor 210 under the control of a controller 216. Similar to the description related to Fig. 1, the centrifugal separation chamber 206 can often be part of a disposable portion, sometimes referred to as a disposable flow circuit, that comes into more direct contact with the biological fluids that are being processed by the device, which can correspondingly be swapped in/out and/or changed to help minimize and/or avoid cross contamination relative to multiple different fluid processing jobs.

Together, the centrifuge compartment 204 and the centrifugal separation chamber can be considered to form an isolated mass 220, which in at least some instances may be monitored by one or more accelerometers 222 to help identify the presence of a condition that might be symptomatic of the presence of a potential problem. Furthermore, it may be possible to separately monitor and/or test one or more vector components of the acceleration being monitored, so as to isolate the acceleration relative to one or more directions, such as one or more orthogonal directions in 3D space, related to one or more of an X, Y and Z direction. Each one of these directions, could have a respective acceleration threshold, which if exceeded could be used to identify the possibility of a problem.

In some cases, the acceleration check could be performed following a fluid prime after an initial spin up. Such a test could be (1) a one time test, (2) a test that is performed continuously, and/or (3) a test that is performed multiple times at specific speeds during the spin up of the centrifuge. Still further possible conditions could be used to trigger one or more related tests without departing from the teachings of the present application. Once the relevant threshold being tested is detected as having been exceeded an alert can be generated, which could be indicative of an improper loading of the centrifugal separation chamber.

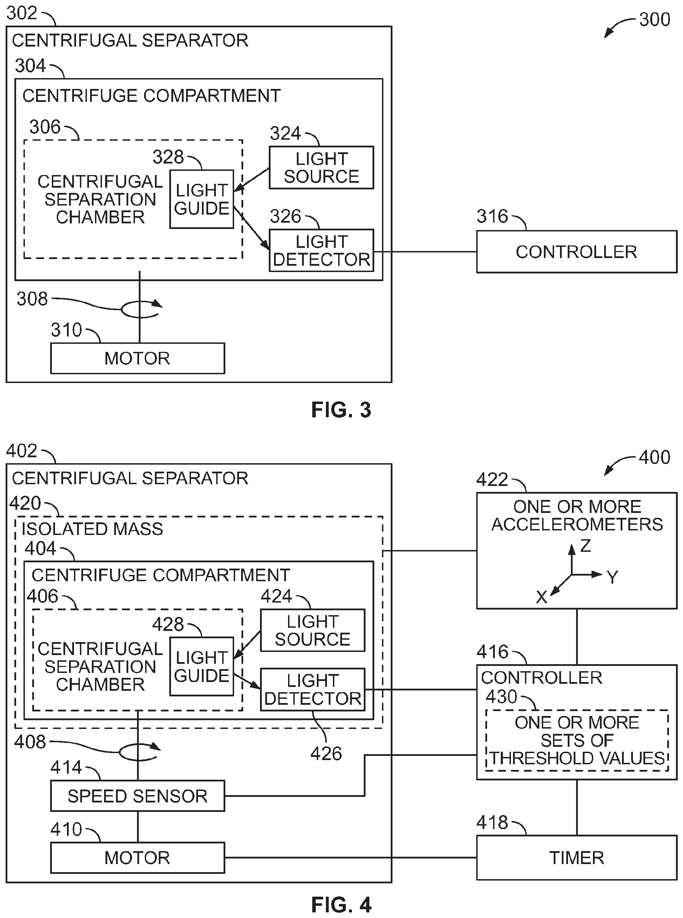

Fig. 3 illustrates a partial block diagram 300 of a biological fluid processing device for use in detecting the proper loading of a centrifugal separation chamber 306, in accordance with at least a still further aspect of the present application. In the illustrated embodiment, the biological fluid processing device includes a centrifugal separator 302, which has a centrifuge compartment 304, which is adapted for receiving a centrifugal separation chamber 306, and spinning 308 the same by a motor 310 under the control of a controller 316. Similar to the description related to Figs. 1 and 2, the centrifugal separation chamber 306 is typically part of a disposable portion, sometimes referred to as a disposable flow circuit, that comes into more direct contact with the biological fluids that are being processed by the device.

In the illustrated embodiment, the centrifuge compartment can include a light source 324 and light detector 326, which are arranged to interact with a light guide 328 included as part of the centrifugal separation chamber 306, at one of a plurality of potential relative rotational orientations, as the centrifugal separation chamber 306 is spun 308 by the motor 310. Light source 324 and light detector 326 are part of the interface detection system (described in greater detail below) included in the blood separation device for detecting and monitoring the quality of the separation of the blood components such as the separation of red blood cells and plasma (platelet-rich plasma), as described for example in U.S. Patent no. 12,214,366, the contents of which are incorporated herein by reference. At this one particular rotational orientation, a first end of the light guide 328 is rotationally aligned with the light source 324, and a second end of the light guide 328 is rotationally aligned with the light detector 326, such that light received from the light source 324 at the first end is conveyed internally within the light guide 328, and is emitted at the second end so as to be detected by the rotationally aligned light detector 326. When the light detector 326 receives light from the light source 324 via the light guide 328 a change in the state of the light detector 326 causes an interface signal to be generated.

In absence of the rotational alignment of the light guide 328, relative to the light source 324 and the light detector 326, light which is emitted by the light source 324 does not have a propagation path which would allow it to be received by the light detector 326. Correspondingly, in absence of a correctly loaded centrifugal separation chamber 306, which is sufficiently spinning so as to be at least periodically rotationally aligned, so as to allow for a light path between the light source 324 and the light detector 326 to periodically exist, would result in the light detector 326 that fails to produce an interface signal. Correspondingly, by analyzing the presence and the nature of the interface signal (i.e. the generated shape or characteristics), it may be possible to identify a presence of the centrifugal separation chamber 306 within the centrifuge compartment 304, the loading status, as well as potentially the rate at which the centrifugal separation chamber 306 might be spinning. To the extent that the registration of the alignment of the light source 324 and the light detector 326 relative to the respective ends of the light guide 328 may be affected by an improper loading condition of the centrifugal separation chamber 306, detection of an improper loading condition via analysis of the characteristics of the interface signal by the controller 316 may further be possible. For example, a misloading of the centrifugal separation chamber could cause the interface signal to exhibit a signal that has a pulse width or peak to peak signal value, that deviates from expected norms, which could be symptomatic of an improper loading of the centrifugal separation chamber.

Fig. 4 illustrates a partial block diagram 400 of a biological fluid processing device for use in detecting the proper loading of a centrifugal separation chamber 406 into a centrifuge compartment 404 of a centrifugal separator 402, which incorporates various features from the aspects illustrated in each of Figs. 1, 2 and 3. Such features include the speed sensor 414 and timer 418 of Fig. 1; the one or more accelerometers 422 of Fig. 2; and the light source 424, light detector 426, and the light guide 428 of Fig. 3, which can selectively provide information to the controller 416, which can then be used for identifying the presence of a misloaded centrifugal separation chamber 406, that might impact the ability of the motor 410 to rotate or spin 408, the centrifugal separation chamber 406. Similar to Fig. 2, the centrifuge compartment 404 together with the centrifugal separation chamber 406, mounted thereon, can form an isolated mass 420. In turn, the isolated mass 420 can interact with the one or more accelerometers 422, while the centrifugal chamber 406 is being spun by the motor, in order to produce a signal representative of a possible acceleration in one or more directions, which can be used by the controller 416 to identify a potential misbalance indicative of a potential improper loading of the centrifugal separation chamber 406.

The controller 416 may be or be a part of a computer having an operating system, which executes apparatus operation software that may be written in a programming language. Any such software could be stored in a related memory, which could be separate from or integrated as part of the controller, or could alternatively be stored elsewhere in the biological fluid processing device. The controller 416 may also use hardware to implement the disclosed operations. For example, the controller 416 may be any programmable processor. Disclosed embodiments may also be implemented on a general-purpose or special purpose computer, a programmed microprocessor or microcontroller, a peripheral integrated circuit element, an application-specific integrated circuit or other integrated circuits, hardware/electronic logic circuits, such as a discrete element circuit, a programmable logic device, such as a programmable logic array, field programmable gate-array, or the like. In general, the controller 416, may be any controller or processor device or devices capable of operating an apparatus and implementing the disclosed embodiments. The controller 416 can further make use of one or more sets of threshold values 430, which could be selectively applied dependent upon a determination of the particular circumstances related to the operation of the biological fluid processing device.

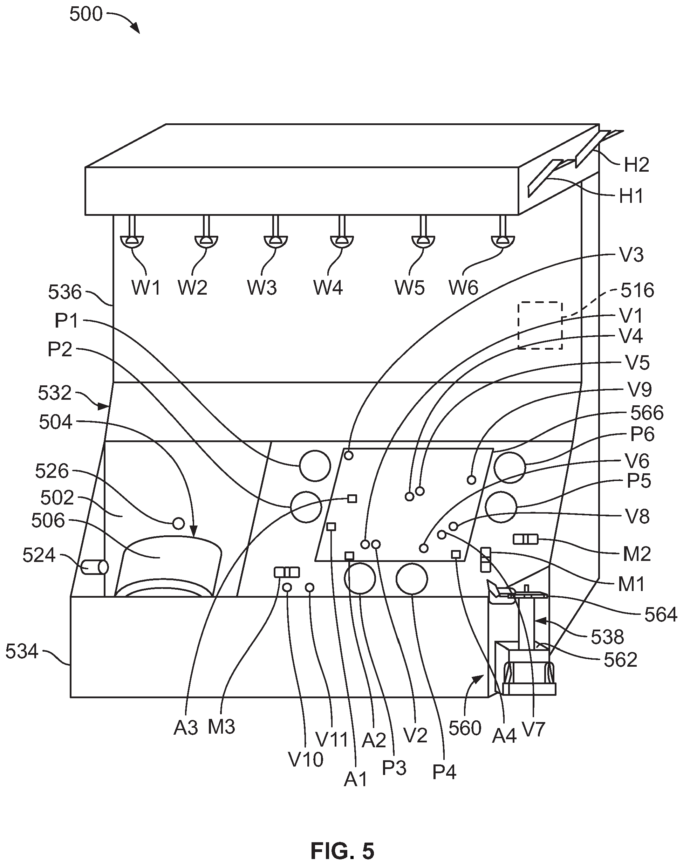

In at least some instances, the biological fluid processing device can be used to process blood. One such example is illustrated in Fig. 5. More specifically, Fig. 5 illustrates a perspective view of an exemplary blood processing device 500 within which the detection of proper loading of a centrifugal separation chamber can be implemented.

The blood processing device 500 is configured as a durable item that is capable of long-term use. It should be understood that the blood processing device 500 is merely exemplary of one possible configuration and that the biological fluid processing devices according to the present disclosure may be configured differently.

In the illustrated embodiment, the blood processing device 500 is embodied in a single housing or case 532. The illustrated case 532 includes a generally horizontal portion 534 (which may include an inclined or angled face or upper surface for enhanced visibility and ergonomics) and a generally vertical portion 536. A spinning membrane separator drive unit 538 and the centrifugal separator 502 are shown as being incorporated into the generally horizontal portion 534 of the case 532, while a controller 516 is shown as being incorporated into the generally vertical portion 536. The configuration and operation of the spinning membrane separator drive unit 538, the centrifugal separator 502, the controller 516, and selected other components of the blood processing device 500 will be described in greater detail.

In the illustrated embodiment, the generally horizontal portion 534 is intended to rest on an elevated, generally horizontal support surface (e.g., a countertop or a tabletop), but it is also within the scope of the present disclosure for the case 532 to include a support base to allow the case 532 to be appropriately positioned and oriented when placed onto a floor or ground surface. It is also within the scope of the present disclosure for the case 532 to be mounted to a generally vertical surface (e.g., a wall), by either fixedly or removably securing the generally vertical portion 536 of the case 532 to the surface.

The case 532 may be configured to assume only the position or configuration of Fig. 5 or may be configured to move between two or more positions or configurations. For example, in one embodiment, the generally horizontal and vertical portions 534 and 536 are joined by a hinge or pivot, which allows the case 532 to be moved between a functional or open configuration (Fig. 5) in which the generally vertical portion 536 is oriented at approximately 90 degrees to the generally horizontal portion 534 and a transport or closed configuration in which the generally vertical portion 536 is rotated about the hinge to approach the generally horizontal portion 534. In such a reconfigurable embodiment, the generally vertical portion 536 may be considered to be the lid of the case 532, while the generally horizontal portion 534 may be considered to be the base. If the case 532 is so reconfigurable, then it may include a latch for releasably locking the case 532 in its closed configuration and/or a handle, which the operator can grasp for transporting the case 532 in its closed configuration.

While it may be advantageous for the blood separation device 500 to be embodied in a compact, portable case 532, it is also within the scope of the present disclosure for the blood separation device to be embodied in a larger case or fixture that is intended to be installed in a single location and remain in that location for an extended period of time. If the blood separation device is provided as a fixture, it may be provided with more components and functionality than a more portable version.

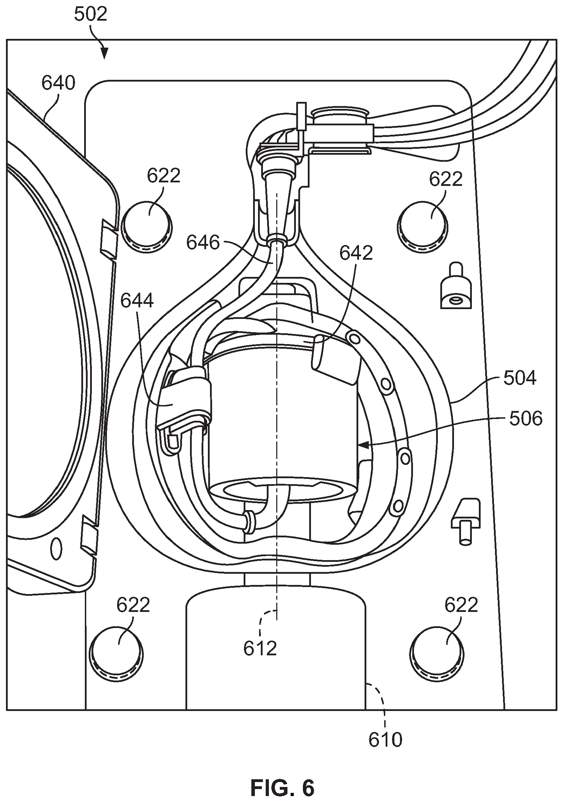

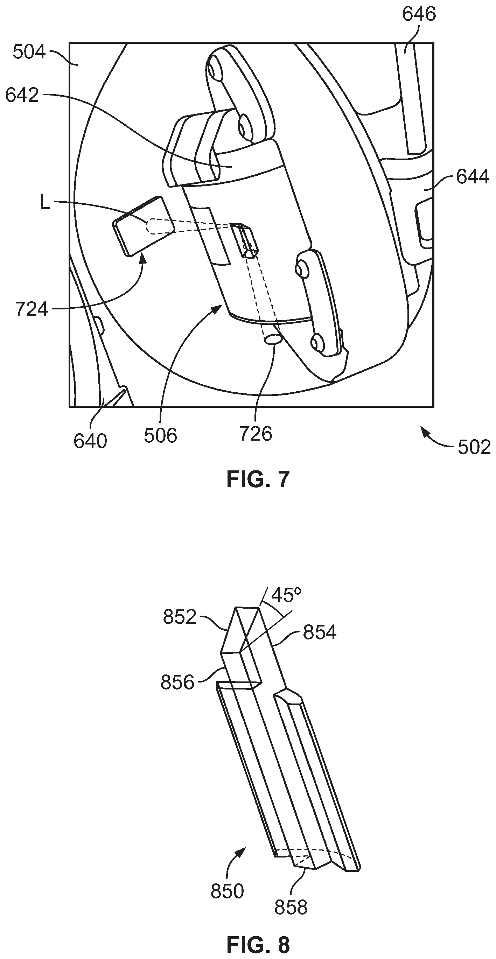

In the illustrated embodiment, the blood processing device 500 includes a centrifugal separator 502, an example of which is illustrated in Figs. 6 and 7. More specifically, Fig. 6 illustrates a partial perspective view of an exemplary centrifugal separator 502, which can be used as part of the exemplary blood processing device 500 illustrated in Fig. 5, and Fig. 7 illustrates a partial perspective view of the exemplary centrifugal separator 502, with a light source and a light detector arranged and oriented to allow for selective light communication between the two via a light guide associated with the centrifugal separation chamber 506 dependent upon the current rotation of the centrifugal separation chamber, relative to a centrifugal separator 502.

The centrifugal separator 502 includes a centrifuge compartment 504 that may receive the other components of the centrifugal separator 502 (Fig. 6). The centrifuge compartment 504 may include a lid 640 that is opened to insert and remove a centrifugal separation chamber 506, which can form a part of a fluid flow circuit, which can include the less permanent and/or more disposable portions of the blood processing device, which can come into more direct contact with the biological fluid being processed and can more readily be changed into and out of the blood processing device 500. During a separation procedure, the lid 640 may be closed with the centrifugal separation chamber 506 positioned within the centrifuge compartment 504, as the centrifugal separation chamber 506 is spun or rotated about an axis 612 under the power of a motor or rotor 610, such as an electric drive motor of the centrifugal separator 502.

The partial perspective view of Fig. 6 further illustrates multiple isolator covers 622, which are each respectively associated with an isolator, at least some of which can incorporate an accelerometer 422, which are positioned between an isolated mass and the rest of the device. More specifically, such as in the illustrated embodiment, the isolator can be positioned underneath the centrifuge compartment 504 (i.e. bucket) and connected to the frame. As previously discussed, the isolated mass can include the combination of the centrifuge compartment 504 and the centrifugal separation chamber 506. While the multiple isolators and correspondingly any of the associated accelerometers 422 (see Fig. 4), are described as being more directly associated with an isolated mass, it is possible that the isolators could be alternatively located, so as to be positioned at the bottom of one or more feet connecting the device to the table or the floor, or could be alternatively positioned relative to the centrifuge arm/chamber, itself, for purposes of detecting a relative acceleration. In such an instance, the elements considered as being included as part of the isolated mass could change, where in some instances the isolated mass could be the entire device or instrument, depending upon the location of the isolators.

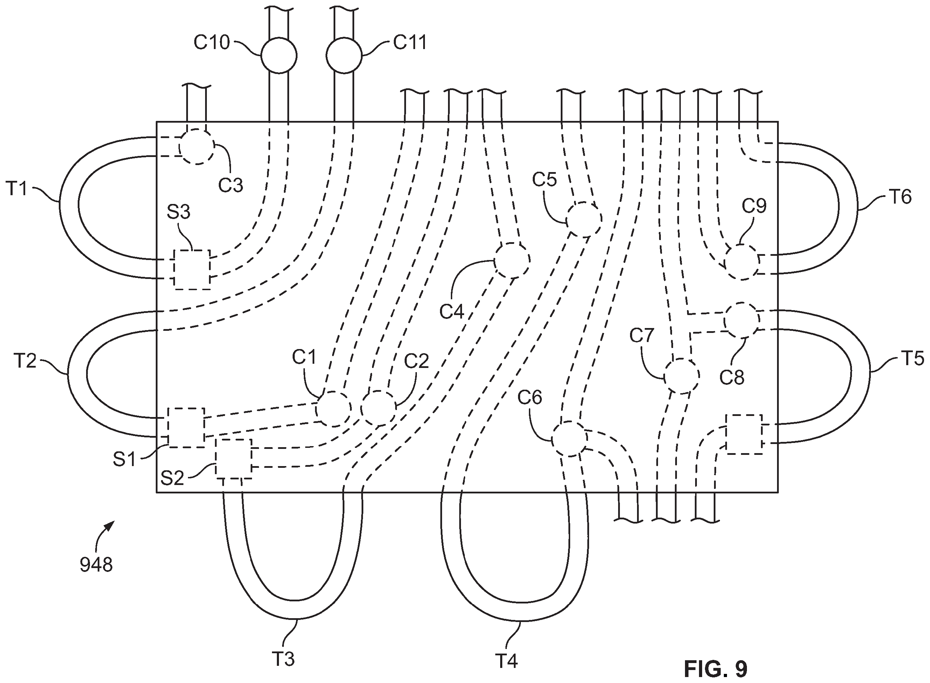

The particular configuration and operation of the centrifugal separator 502 depends upon the particular configuration of the centrifugal separation chamber 506, which, as noted above, can form a part of the fluid flow circuit. In one embodiment, the centrifugal separator 502 is similar in structure and operation to that of the ALYX system manufactured by Fenwal, Inc. of Lake Zurich, Illinois, which is an affiliate of Fresenius Kabi AG of Bad Homburg, Germany, as described in greater detail in U.S. Patent No. 8,075,468, which is incorporated herein by reference. More particularly, the centrifugal separator 502 may include a carriage or support 642 that holds the centrifugal separation chamber 506 and a yoke member 644. The yoke member 644 engages an umbilicus 646 of the fluid flow circuit, which extends between the centrifugal separation chamber 506 and a cassette 948 of the fluid flow circuit (Fig. 9). The yoke member 644 causes the umbilicus 646 to orbit around the centrifugal separation chamber 506 at a one omega rotational speed. The umbilicus 646 twists about its own axis as it orbits around the centrifugal separation chamber 506. The twisting of the umbilicus 646 about its axis as it rotates at one omega with the yoke member 644 imparts a two omega rotation to the centrifugal separation chamber 506, according to known design. The relative rotation of the yoke member 644 at a one omega rotational speed and the centrifugal separation chamber 506 at a two omega rotational speed keeps the umbilicus 646 untwisted, avoiding the need for rotating seals.

Blood is introduced into the centrifugal separation chamber 506 by the umbilicus 646, with the blood being separated (e.g., into a layer of less dense components, such as platelet-rich plasma, and a layer of more dense components, such as packed red blood cells) within the centrifugal separation chamber 506 as a result of centrifugal forces as it rotates. Components of an interface monitoring system may be positioned within the centrifuge compartment 504 to oversee separation of blood within the centrifugal separation chamber 506. As shown in Fig. 7, the interface monitoring system may include a light source 724 and a light detector 726, which is positioned and oriented to receive at least a portion of the light emitted by the light source 724. Preferably, the light source 724 and the light detector 726 are positioned on stationary surfaces of the centrifuge compartment 504, but it is also within the scope of the present disclosure for one or both to be mounted to a movable component of the centrifugal separator 502 (e.g., to the yoke member 644, which rotates at a one omega speed).

The orientation of the various components of the interface monitoring system depends at least in part on the particular configuration of the centrifugal separation chamber 506. In general, though, the light source 724 emits a light beam (e.g., a laser light beam) through the separated blood components within the centrifugal separation chamber 506 (which may be formed of a material that substantially transmits the light or at least a particular wavelength of the light without absorbing it). A portion of the light reaches the light detector 726, which transmits an interface signal to the controller 516 that is indicative of the location of an interface between the separated blood components. If the controller 516 determines that the interface is in the wrong location (which can affect the separation efficiency of the centrifugal separator 502 and/or the quality of the separated blood components), then it can issue commands to the appropriate components of the blood processing device 500 to modify their operation, so as to move the blood component interface to the proper and/or more desired location. The interface signal may additionally have observable characteristics, from which a determination of the proper loading of the centrifugal separation chamber may be derived.

In the illustrated embodiment, the light source 724 of the interface monitoring system is secured to a fixture or wall of the centrifuge compartment 504 and oriented to emit a light that is directed toward the rotational axis 612 of the centrifugal separator 502, as shown in Fig. 7. If the light detector 726 is positioned at an angle with respect to the light source 724 (as in the illustrated embodiment), the light L emitted by the light source 724 must be redirected from its initial path before it will reach the light detector 726. In the embodiment illustrated in Fig. 8, the light L is redirected by a reflector 852 associated with a light guide 850. The light guide 850, which can include the reflector 852 may be a separate piece that is secured to an inner side wall portion of the centrifugal separation chamber 506 (e.g., by being bonded thereto) or may be integrally formed with the body of the centrifugal separation chamber 506.

In one embodiment, the reflector may be a reflective surface, such as a mirror, that is oriented (e.g., at a 45° angle) to direct light L emitted by the light source 724 to the light detector 726. In the illustrated embodiment, the light guide 850 can be formed of a light-transmissive material (e.g., a clear plastic material) and has inner and outer walls 854 and 856 and first and second end walls 852 and 858. The inner wall 854 is positioned against the inner side wall portion of the centrifugal separation chamber 506 and is oriented substantially perpendicular to the initial path of the light L from the light source 724. This allows light L from the light source 724 to enter into the light guide 850 via the inner wall 854 while continuing along its initial path. The light L continues through the light guide 850 along its initial path until it encounters the first end wall 852. The first end wall 852 is oriented at an angle (e.g., an approximately 45° angle) with respect to the first surface 854 and the second end wall 858, causing the light to be redirected within the light guide 850, rather than exiting the light guide 850 via the first end wall 852.

The first end wall 852 directs the light L at an angle to its initial path (which may be an approximately 90° angle, directing it from a path toward the rotational axis 612 to a path that is generally parallel to the rotational axis 612) toward the second end wall 858. The first end wall 852 and the inner and outer walls 854 and 856 of the light guide 850 may be configured to transmit the redirected light L from the first end wall 852 to the second end wall 858 by total internal reflection. The second end wall 858 is oriented substantially perpendicular to the redirected path of the light L through the light guide 850, such that the light L will exit the light guide 850 via the second end wall 858, continuing along its redirected path. In one embodiment, the second end wall 858 is roughened or textured or otherwise treated or conditioned to diffuse the light L as the light exits the light guide 850, which may better ensure that the light L reaches the light detector 726.

In the illustrated embodiment, the blood processing device 500, in addition to including the centrifugal separator 502, also includes a spinner support or spinning membrane separator drive unit 560 (Fig. 5) for accommodating a generally cylindrical spinning membrane separator 538 of the fluid flow circuit. U.S. Patent No. 5,194,145 describes an exemplary spinning membrane separator drive unit that would be suitable for incorporation into the blood processing device 500, but it should be understood that the spinning membrane separator drive unit 560 may be differently configured without departing from the scope of the present disclosure.

The illustrated spinning membrane separator drive unit 560 has a base 562 configured to receive a lower portion of the spinning membrane separator 538 and an upper end cap 564 to receive an upper portion of the spinning membrane separator 538. Preferably, the upper end cap 564 is positioned directly above the base 562 to orient a spinning membrane separator 538 received by the spinning membrane separator drive unit 560 vertically and to define a vertical axis about which the spinning membrane separator 538 is spun. While it may be advantageous for the spinning membrane separator drive unit 560 to vertically orient the spinning membrane separator 538, it is also within the scope of the present disclosure for the spinning membrane separator 538 to be differently oriented when mounted to the blood processing device 500. The spinning membrane separator allows for an alternative approach for potentially separating a bodily fluid into its constituent components.

The generally horizontal portion 534 of the case 532 of the illustrated blood separation device 500 includes a cassette station 566, which accommodates a cassette 948 of the fluid flow circuit (Fig. 9). In one embodiment, the cassette station 566 is similarly configured to the cassette station of U.S. Patent No. 5,868,696 (which is incorporated herein by reference), but can be adapted to include additional components and functionality. The illustrated cassette station 566 includes a plurality of clamps or valves V1-V9 (Fig. 5), which move between a plurality of positions (e.g., between a retracted or lowered position and an actuated or raised position) to selectively contact or otherwise interact with corresponding valve stations C1-C9 of the cassette 948 of the fluid flow circuit. Depending on the configuration of the fluid flow circuit, its cassette 948 may not include a valve station C1-C9 for each valve V1-V9 of the cassette station 566, in which case fewer than all of the valves V1-V9 may be used in any related separation procedure.

In the actuated position, a valve V1-V9 engages the associated valve station C1-C9 to prevent fluid flow through that valve station C1-C9 (e.g., by closing one or more ports associated with the valve station C1-C9, thereby preventing fluid flow through that port or ports). In the retracted position, a valve V1-V9 is disengaged from the associated valve station C1-C9 (or less forcefully contacts the associated valve station C1-C9 than when in the actuated position) to allow fluid flow through that valve station C1-C9 (e.g., by opening one or more ports associated with the valve station C1-C9, thereby allowing fluid flow through that port or ports). Additional clamps or valves V10 and V11 may be positioned outside of the cassette station 566 to interact with portions or valve stations C10 and C11 (which may be lengths of tubing) of the fluid flow circuit to selectively allow and prevent fluid flow therethrough. The valves V1-V9 and corresponding valve stations C1-C9 of the cassette station 566 and cassette 948 may be differently configured and operate differently from the valves V10 and V11 and valve stations C10 and C11 that are spaced away from the cassette station 566.

The cassette station 566 may be provided with additional components, such as pressure sensors A1-A4, which interact with sensor stations S1-S4 of the cassette 948 to monitor the pressure at various locations of the fluid flow circuit. For example, if the blood source is a human donor, one or more of the pressure sensors A1-A4 may be configured to monitor the pressure of the donor's vein during blood draw and return. Other pressure sensors A1-A4 may monitor the pressure of the spinning membrane separator 538 and the centrifugal separation chamber 506. The controller 516 may receive signals from the pressure sensor A1-A4 that are indicative of the pressure within the fluid flow circuit and, if a signal indicates a low- or high-pressure condition, the controller 516 may initiate an alarm or error condition to alert an operator to the condition and/or to attempt to bring the pressure to an acceptable level without operator intervention.

The blood processing device 500 may also include a plurality of pumps P1-P6 to cause fluid to flow through the fluid flow circuit. The pumps P1-P6 may be differently or similarly configured and/or function similarly or differently from each other. In the illustrated embodiment, the pumps P1-P6 are configured as peristaltic pumps, which may be generally configured as described in U.S. Patent No. 5,868,696. Each pump P1-P6 engages a different tubing loop T1-T6 extending from a side surface of the cassette 948 (Fig. 9) and may be selectively operated under command of the controller 516 to cause fluid to flow through a portion of the fluid flow circuit. In one embodiment, all or a portion of the cassette station 566 may be capable of translational motion in and out of the case 532 to allow for automatic loading of the tubing loops T1-T6 into the associated pump P1-P6.

The illustrated blood processing device 500 also includes a spinner inlet sensor M1 for determining one or more properties of a fluid flowing into a spinning membrane separator 538 mounted within the spinning membrane separator drive unit 560. The illustrated blood processing device 500 further includes a spinner outlet sensor M2, which accommodates tubing of the fluid flow circuit that flows a separated blood component out of the spinning membrane separator 538. The spinner outlet sensor M2 monitors the fluid to determine one or more properties of the fluid,and may do so by optically monitoring the fluid as it flows through the tubing or by any other suitable approach. This may be done using an optical monitor of the type described in U.S. Patent No. 8,556,793 (which is incorporated herein by reference) or by any other suitable device and/or method.

The illustrated blood processing device 500 also includes an air detector M3 (e.g., an ultrasonic bubble detector), which accommodates tubing of the fluid flow circuit that flows fluid to a recipient. It may be advantageous to prevent air from reaching the recipient, whether a human recipient (e.g., the same human that serves as the blood source) or a non-human recipient (e.g., a storage bag or container), so the air detector M3 may transmit signals to the controller 516 that are indicative of the presence or absence of air in the tubing. If the signal is indicative of air being present in the tubing, the controller 516 may initiate an alarm or error condition to alert an operator to the condition and/or to take corrective action to prevent the air from reaching the recipient (e.g., by reversing the flow of fluid through the tubing or diverting flow to a vent location).

The generally vertical portion 536 of the case 532 may include a plurality of weight scales W1-W6 (six are shown, but more or fewer may be provided), each of which may support one or more fluid containers of the fluid flow circuit (not shown). The containers, in some instances, can receive blood components separated during processing or intravenous fluids or additive fluids. Each weight scale W1-W6 transmits to the controller 516 a signal that is indicative of the weight of the fluid within the associated container to track the change of weight during the course of a procedure. This allows the controller 516 to process the incremental weight changes to derive fluid processing volumes and flow rates and subsequently generate signals to control processing events based, at least in part, upon the derived processing volumes.

The illustrated case 532 is also provided with a plurality of hooks or supports H1 and H2 that may support various components of the fluid flow circuit or other suitably sized and configured objects.

In one embodiment, the controller 516 may be mounted inside the generally vertical portion 536 of the case 532, adjacent to or incorporated into an operator interface station (e.g., a touchscreen). In other embodiments, the controller 516 and operator interface station may be associated with the generally horizontal portion 534 or may be incorporated into a separate device that is connected (either physically, by a cable or the like, or wirelessly) to the blood processing device 500.

If the operation of any of the components is outside of an acceptable range, then the controller 516 may initiate an alarm or error condition to alert the operator and/or take action to attempt to correct the condition. The appropriate corrective action will depend upon the particular error condition and may include action that is carried out with or without the involvement of an operator.

If provided, an operator interface station associated with the controller 516 allows the operator to view on a screen or display (in alpha-numeric format and/or as graphical images) information regarding the operation of the system. The operator interface station also allows the operator to select applications to be executed by the controller 516, as well as to change certain functions and performance criteria of the system. If configured as a touchscreen, the screen of the operator interface station can receive input from an operator via touch-activation. Otherwise, if the screen is not a touchscreen, then the operator interface station may receive input from an operator via a separate input device, such as a computer mouse or keyboard. It is also within the scope of the present disclosure for the operator interface station to receive input from both a touchscreen and a separate input device, such as a keypad.

As for the fluid flow circuit, it is intended to be a sterile, single use, disposable item. Before beginning a given blood processing and/or collection procedure, the operator loads various components of the fluid flow circuit into the case 532 in association with the blood processing device 500. Generally, the centrifugal separation chamber 506 and the spinning membrane separator 538 would be included as part of the fluid flow circuit, and therefore they could be frequently loaded and reloaded, where after each such instance, it is possible that the element may have been improperly loaded. Relative to the cassette 948, illustrated in Fig. 9, any centrifugal separation chamber 506 and any spinning membrane separator 538 could be coupled to the cassette via any one of the illustrated discontinued tubes. In at least the case of the centrifugal separation chamber 506, the centrifugal separation chamber 506 may be coupled to the cassette 948 via an umbilicus 646. Other ones of the illustrated discontinued tubes could be coupled to one or more collection containers or bags including one or more whole or separated blood components and/or one or more additive solutions.

In at least one embodiment, the cassette 948 is similarly configured to the cassette of U.S. Patent No. 5,868,696, but is adapted to include additional components (e.g., more tubing loops T1-T6) and functionality.

The discussed check regarding the proper loading of the centrifugal separation chamber within the centrifugal separator could be performed as part of an integrity check, wherein the controller attempts to ensure that the various components are properly connected and functioning.

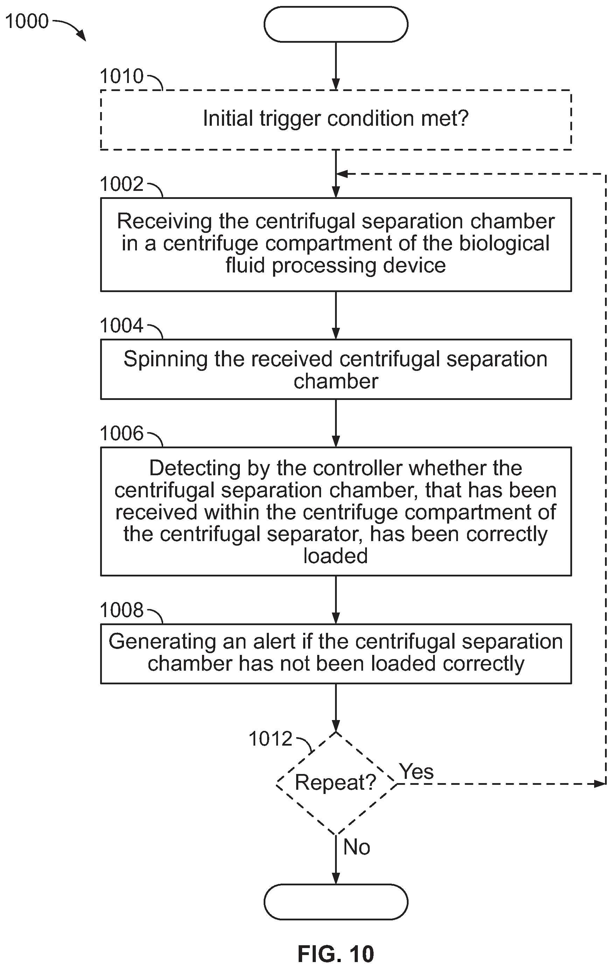

Fig. 10 illustrates a flow diagram of a method 1000 for detecting the proper loading of a centrifugal separation chamber in a centrifugal separator. The method includes receiving 1002 the centrifugal separation chamber in a centrifuge compartment of the biological fluid processing device. The received centrifugal separation chamber is then spun 1004. A detection 1006 is then made by the controller, as to whether the centrifugal separation chamber, that has been received within the centrifuge compartment of the centrifugal separator, has been correctly loaded. An alert is generated 1008, if the centrifugal separation chamber has been detected as having not been loaded correctly.

In at least some embodiments, an optional check as to whether an initial trigger condition has been met 1010 can serve as a precursor to determining whether the centrifugal separation chamber has been properly loaded. At least one such initial trigger condition can include a determination as to whether the end of a fluid prime has occurred. Further, an initial trigger condition check could additionally and/or alternatively include the detection of an opening and subsequent closing of the centrifuge door during a stop, as the centrifugal separation chamber may have been unloaded and reloaded by the operator during this time.

In some instances, the determination of a proper loading of the centrifugal separation chamber could be a one time check. In other instances, the check could be performed continuously. In further instances, the check could be performed periodically. In still further instances, the check could be performed multiple times at specific speeds during the spin up of the centrifuge. The various possibilities related to the potential repeating of the determination of proper loading could be optionally decided as part of a repeat determination 1012.

In some instances, it may be possible to perform a check at an initial intermediate stage, such as the determination of proper loading could be completed with a dry kit prior to all of the fluids having been connected. By testing the proper loading with a dry kit, the saving of saline and one or more additives is possible, where if the centrifugal separation chamber had been improperly loaded and the consequences of the misloading had been severe, the breakage and associated fluid spillage might have been avoided. However, testing with a dry kit or other adjustments of testing conditions, may result in the need to involve a selection of a different set of thresholds including alert thresholds and/or acceptance criteria for purposes of determining an improper loading.

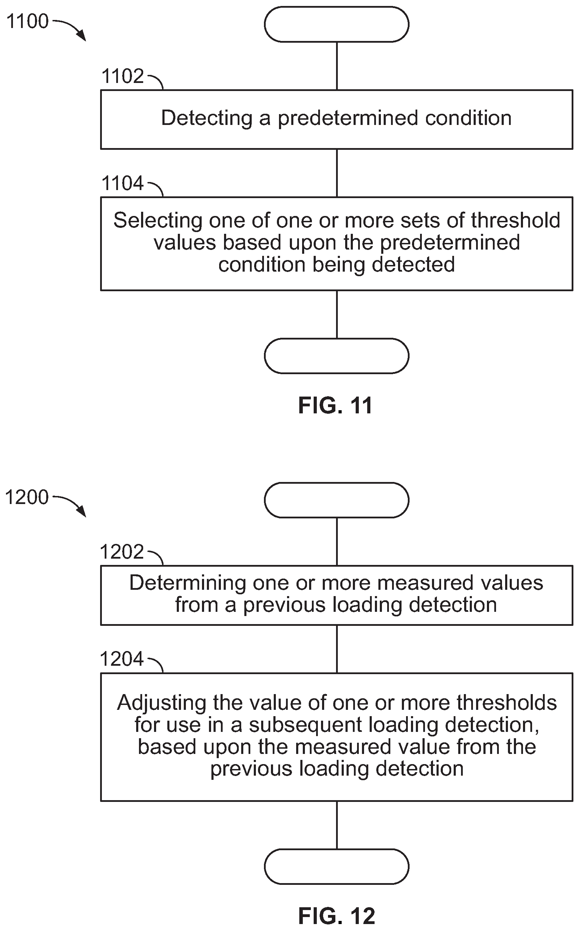

Fig. 11 illustrates a flow diagram 1100 of a method for selecting one of one or more sets of threshold values in connection with detecting the proper loading of a centrifugal separation chamber. The method includes detecting 1102 a predetermined condition or a variance related to the conditions associated with the determination of a proper loading, and selecting 1104 one of one or more sets of threshold values based upon the predetermined condition or variance having been detected.

It is further possible that the biological fluid processing device could take the results of prior testing and use the results to adjust the value of one or more thresholds used in a detection associated with a subsequent loading, based upon the measured value from a previous loading detection. Such a refinement/adjustment of the values used for subsequent loading detections can involve a form of dynamic or machine learning, where it may be possible that past testing could highlight possible idiosyncrasies related to a particular device or a particular type of device, which would allow for measured values during previous testing to be used to adjust the thresholds used in determining whether a subsequent loading had been successful or unsuccessful. For example, the time it takes the centrifuge to reach speed can vary based on the motor, and the biological fluid processing device can account for this by shortening or extending one or more of the testing thresholds, based upon previous successful loads to make the threshold more indicative of the specific instrument hardware related to a particular device. Similarly, the accelerometer thresholds can be adjusted if the balance of the centrifuge is not as good as it relates to a particular device, so as to help prevent possible falsely alerting the operator of a potential misload.

Fig. 12 illustrates a flow diagram 1200 of a method for adjusting the value of one or more thresholds for use in connection with detecting the proper loading of a centrifugal separation chamber. The method includes determining 1202 one or more values from a previous loading detection, and adjusting 1204 the value of one or more threshold values for use in a subsequent loading detection, based upon the measured value from the previous loading detection.

It is possible that the related selection of one of one or more sets of threshold values (Fig. 11), and the possible adjusting of the value of one or more threshold values for use in a subsequent proper/improper loading detection, based upon the measured value from a previous loading detection, could optionally be used either separately or together with the features for detecting the proper loading of a centrifugal separation chamber within a centrifugal separator as outlined in Fig. 10, possibly enhancing the base detection techniques. However, the further incorporation of these features may not be beneficial in all instances.

The methods of this disclosure can be implemented on a programmed processor. However, the controllers and flowcharts may also be implemented on a general purpose or special purpose computer, a programmed microprocessor or microcontroller and peripheral integrated circuit elements, an integrated circuit, a hardware electronic or logic circuit such as a discrete element circuit, a programmable logic device, or the like. In general, any device on which resides a finite state machine capable of implementing the flowcharts shown in the figures may be used to implement the controller functions of this disclosure.

Aspects of the present subject matter described above may be beneficial alone or in combination with one or more other aspects. Without limiting the foregoing description, in accordance with one aspect of the subject matter herein, there is provided Aspect 1, a biological fluid processing device. The biological fluid processing device includes a centrifugal separator having a centrifuge compartment adapted for receiving a centrifugal separation chamber, wherein the centrifugal separator is further adapted for spinning the received centrifugal separation chamber. The biological fluid processing device further includes a controller configured for detecting whether any centrifugal separation chamber, that has been received within the centrifuge compartment of the centrifugal separator, has been correctly loaded, and generating an alert if the centrifugal separation chamber has not been correctly loaded.

In accordance with Aspect 2, which may be used or combined with Aspect 1, the centrifugal separator further has a motor coupled to the received centrifugal separation chamber for spinning the received centrifugal separation chamber, where the motor is associated with a rotational speed sensor coupled to the controller for detecting a speed at which the centrifugal separation chamber is currently spinning, and a timer for respectively associating the detected speeds with each of one or more reference points in time.

In accordance with Aspect 3, which may be used or combined with Aspect 2, the controller determines a time duration for which it takes the motor of the centrifugal separator upon initiating the spinning of the received centrifugal separation chamber to reach a predetermined slow spin up testing speed, wherein the alert indicating an incorrectly loaded centrifugal separation chamber is produced when the time duration for which it takes the motor to reach the predetermined slow spin up testing speed exceeds a predetermined slow spin up time duration threshold.

In accordance with Aspect 4, which may be used or combined with Aspect 3, the predetermined slow spin up testing speed is less than the anticipated full speed of spinning of the centrifugal separation chamber during normal operation.

In accordance with Aspect 5, which may be used or combined with any one of Aspects 2 through 4, when the controller determines that the speed detected by the speed sensor fails to exceed a predetermined spin up failure testing speed within a predetermined spin up failure testing time duration after initiating the spinning of the received centrifugal separation chamber, the alert indicating the incorrectly loaded centrifugal separation chamber is produced.

In accordance with Aspect 6, which may be used or combined with any one of Aspects 2 through 5, the controller determines a time duration for which it takes the motor of the centrifugal separator upon initiating the spinning of the received centrifugal separation chamber to reach a predetermined too fast spin up testing speed, wherein the alert indicating an incorrectly loaded centrifugal separation chamber is produced when the time duration for which it takes the motor to reach the predetermined too fast spin up testing speed is less than a predetermined too fast spin up time duration threshold.

In accordance with Aspect 7, which may be used or combined with any one of Aspects 2 through 6, the controller is communicatively coupled to one or more accelerometers associated with any detectable acceleration of at least one of the centrifuge compartment and the centrifugal separation chamber during the spinning by the motor.

In accordance with Aspect 8, which may be used or combined with Aspect 7, the one or more accelerometers measure an amount of acceleration in each of multiple directions, which can be separately monitored and compared with one or more respective threshold values, and wherein if the measured amount of acceleration in any one of the multiple directions exceeds at least one of the one or more respective threshold values, then the alert indicating an incorrectly loaded centrifugal separation chamber is produced.

In accordance with Aspect 9, which may be used or combined with Aspect 8, the measuring of an amount of acceleration in each of multiple directions includes a determination of an amount of acceleration in each of three orthogonal directions.

In accordance with Aspect 10, which may be used or combined with any one of Aspects 7 through 9, the amount of acceleration measured by the one or more accelerometers is analyzed by the controller after a fluid prime.

In accordance with Aspect 11, which may be used or combined with any one of Aspects 7 through 10, the amount of acceleration measured by the one or more accelerometers is analyzed by the controller after an initial spin up of the centrifugal separation chamber.

In accordance with Aspect 12, which may be used or combined with any one of Aspects 7 through 11, the amount of acceleration measured by the one or more accelerometers is continuously analyzed by the controller during the spinning of the received centrifugal separation chamber by the motor.

In accordance with Aspect 13, which may be used or combined with any one of Aspects 7 through 11, the amount of acceleration measured by the one or more accelerometers is periodically analyzed by the controller during the spinning of the received centrifugal separation chamber by the motor.

In accordance with Aspect 14, which may be used or combined with any one of Aspects 7 through 11, the amount of acceleration measured by the one or more accelerometers is analyzed by the controller at each of a predetermined set of times as determined by the timer after initiation of the spin up during the spinning of the received centrifugal separation chamber by the motor.

In accordance with Aspect 15, which may be used or combined with any one of Aspects 7 through 11, the amount of acceleration measured by the one or more accelerometers is analyzed by the controller at each of a predetermined set of speeds being reached as detected by the speed sensor after initiation of the spin up during the spinning of the received centrifugal separation chamber by the motor.

In accordance with Aspect 16, which may be used or combined with any one of Aspects 1 through 15, the biological fluid processing device further includes a light source and light detector, which are communicatively coupled for producing an interface signal during a subset of the relative rotational positions of the centrifuge compartment and the received centrifugal separation chambers during which a light guide associated with the centrifugal separation chamber has a first end at least partially aligned with the light source and a second end at least partially aligned with the light detector.

In accordance with Aspect 17, which may be used or combined with Aspect 16, a presence of the interface signal is used by the controller to detect whether the centrifugal separation chamber, which has been received within the centrifuge compartment of the centrifugal separator, has been correctly loaded.

In accordance with Aspect 18, which may be used or combined with Aspect 16, where the interface signal includes associated detectable characteristics, where the associated detectable characteristics of the interface signal are used by the controller to detect an improper loading of the centrifugal separation chamber within the centrifuge compartment of the centrifugal separator.

In accordance with Aspect 19, which may be used or combined with any one of Aspects 1 through 18, the controller detects whether the centrifugal separation chamber, which has been received within the centrifuge compartment of the centrifugal separator, has been correctly loaded, at the end of fluid prime.

In accordance with Aspect 20, which may be used or combined with any one of Aspects 1 through 19, the controller detects whether the centrifugal separation chamber has been correctly loaded, after a centrifuge door of the centrifuge compartment has been detected as having been opened, and subsequently closed.

In accordance with Aspect 21, which may be used or combined with any one of Aspects 1 through 20, the biological fluid processing device further includes one or more fluid reservoirs, wherein the controller detects whether the centrifugal separation chamber has been correctly loaded, before at least some of the one or more fluid reservoirs have been connected.

In accordance with Aspect 22, which may be used or combined with Aspect 21, the controller adjusts one or more testing values for detecting whether the centrifugal separation chamber has been correctly loaded, based upon the particular fluid reservoirs of the one or more fluid reservoirs that have been connected prior to the detection.

In accordance with Aspect 23, which may be used or combined with any one of Aspects 1 through 22, the controller adjusts one or more subsequent testing values for successively detecting whether the centrifugal separation chamber has been correctly loaded, based upon any measured values associated with any previous successfully detected loadings of the centrifugal separation chambers into the centrifuge compartment.

In accordance with Aspect 24, which may be used or combined with Aspect 23, the adjustment of the one or more subsequent testing values are determined based upon the measured values from previous successfully detected loadings related to a particular biological fluid processing device.

In accordance with Aspect 25, which may be used or combined with any one of Aspects 1 through 24, the biological fluid processing device further includes an alarm coupled to the controller for generating the alert when the controller detects that the centrifugal separation chamber has not been correctly loaded into the centrifuge compartment.

In accordance with Aspect 26, which may be used or combined with any one of Aspects 1 through 25, the biological fluid processing device is a blood separation device.

In accordance with Aspect 27, which may be used or combined with any one of Aspects 1 through 26, the centrifugal separation chamber, which is adapted to be received by the centrifuge compartment of the centrifugal separator is part of a disposable flow circuit.

In accordance with Aspect 28, a biological fluid processing device is provided. The biological fluid processing device includes a disposable flow circuit, which has a centrifugal separation chamber, and a centrifugal separator having a centrifuge compartment for receiving the centrifugal separation chamber of the disposable flow circuit, wherein the centrifugal separator is adapted for spinning the received centrifugal separation chamber. The biological fluid processing device further includes a controller, which is configured for detecting whether any centrifugal separation chamber, that has been received within the centrifuge compartment of the centrifugal separator, has been correctly loaded, and generating an alert if the centrifugal separation chamber has not been correctly loaded.

In accordance with Aspect 29, which may be used or combined with Aspect 28, the centrifugal separator further has a motor coupled to the received centrifugal separation chamber for spinning the received centrifugal separation chamber, where the motor is associated with a rotational speed sensor coupled to the controller for detecting a speed at which the centrifugal separation chamber is currently spinning, and a timer for respectively associating the detected speeds with each of one or more reference points in time.

In accordance with Aspect 30, which may be used or combined with Aspect 29, the controller determines a time duration for which it takes the motor of the centrifugal separator upon initiating the spinning of the received centrifugal separation chamber to reach a predetermined slow spin up testing speed, and wherein the alert indicating an incorrectly loaded centrifugal separation chamber is produced when the time duration for which it takes the motor to reach the predetermined slow spin up testing speed exceeds a predetermined slow spin up time duration threshold.

In accordance with Aspect 31, which may be used or combined with Aspect 30, the predetermined slow spin up testing speed is less than the anticipated full speed of spinning of the centrifugal separation chamber during normal operation.

In accordance with Aspect 32, which may be used or combined with any one of Aspects 29 through 31, when the controller determines that the speed detected by the speed sensor fails to exceed a predetermined spin up failure testing speed within a predetermined spin up failure testing time duration after initiating the spinning of the received centrifugal separation chamber, the alert indicating the incorrectly loaded centrifugal separation chamber is produced.

In accordance with Aspect 33, which may be used or combined with any one of Aspects 29 through 32, the controller determines a time duration for which it takes the motor of the centrifugal separator upon initiating the spinning of the received centrifugal separation chamber to reach a predetermined too fast spin up testing speed, and wherein the alert indicating an incorrectly loaded centrifugal separation chamber is produced when the time duration for which it takes the motor to reach the predetermined too fast spin up testing speed is less than a predetermined too fast spin up time duration threshold.

In accordance with Aspect 34, which may be used or combined with any one of Aspects 29 through 33, the controller is communicatively coupled to one or more accelerometers associated with any detectable acceleration of at least one of the centrifuge compartment and the centrifugal separation chamber during the spinning by the motor.

In accordance with Aspect 35, which may be used or combined with Aspect 34, the one or more accelerometers measure an amount of acceleration in each of multiple directions, which can be separately monitored and compared with one or more respective threshold values, and wherein if the measured amount of acceleration in any one of the multiple directions exceeds at least one of the one or more respective threshold values, then the alert indicating an incorrectly loaded centrifugal separation chamber is produced.

In accordance with Aspect 36, which may be used or combined with Aspect 35, the measuring of an amount of acceleration in each of multiple directions includes a determination of an amount of acceleration in each of three orthogonal directions.

In accordance with Aspect 37, which may be used or combined with any one of Aspects 34 through 36, the amount of acceleration measured by the one or more accelerometers is analyzed by the controller after a fluid prime.

In accordance with Aspect 38, which may be used or combined with any one of Aspects 34 through 37, the amount of acceleration measured by the one or more accelerometers is analyzed by the controller after an initial spin up of the centrifugal separation chamber.

In accordance with Aspect 39, which may be used or combined with any one of Aspects 34 through 38, the amount of acceleration measured by the one or more accelerometers is continuously analyzed by the controller during the spinning of the received centrifugal separation chamber by the motor.

In accordance with Aspect 40, which may be used or combined with any one of Aspects 34 through 38, the amount of acceleration measured by the one or more accelerometers is periodically analyzed by the controller during the spinning of the received centrifugal separation chamber by the motor.

In accordance with Aspect 41, which may be used or combined with any one of Aspects 34 through 38, the amount of acceleration measured by the one or more accelerometers is analyzed by the controller at each of a predetermined set of times as determined by the timer after initiation of the spin up during the spinning of the received centrifugal separation chamber by the motor.

In accordance with Aspect 42, which may be used or combined with any one of Aspects 34 through 38, the amount of acceleration measured by the one or more accelerometers is analyzed by the controller at each of a predetermined set of speeds being reached as detected by the speed sensor after initiation of the spin up during the spinning of the received centrifugal separation chamber by the motor.

In accordance with Aspect 43, which may be used or combined with any one of Aspects 28 through 42, the biological fluid processing device further includes a light source and light detector, which are communicatively coupled for producing an interface signal during a subset of the relative rotational positions of the centrifuge compartment and the received centrifugal separation chambers during which a light guide associated with the centrifugal separation chamber has a first end at least partially aligned with the light source and a second end at least partially aligned with the light detector.

In accordance with Aspect 44, which may be used or combined with Aspect 43, a presence of the interface signal is used by the controller to detect whether the centrifugal separation chamber, which has been received within the centrifuge compartment of the centrifugal separator, has been correctly loaded.

In accordance with Aspect 45, which may be used or combined with Aspect 43, where the interface signal includes associated detectable characteristics, where the associated detectable characteristics of the interface signal are used by the controller to detect an improper loading of the centrifugal separation chamber within the centrifuge compartment of the centrifugal separator.

In accordance with Aspect 46, which may be used or combined with any one of Aspects 28 through 45, the controller detects whether the centrifugal separation chamber, which has been received within the centrifuge compartment of the centrifugal separator, has been correctly loaded, at the end of fluid prime.

In accordance with Aspect 47, which may be used or combined with any one of Aspects 28 through 46, the controller detects whether the centrifugal separation chamber has been correctly loaded, after a centrifuge door of the centrifuge compartment has been detected as having been opened, and subsequently closed.

In accordance with Aspect 48, which may be used or combined with any one of Aspects 28 through 47, the biological fluid processing device further includes one or more fluid reservoirs, wherein the controller detects whether the centrifugal separation chamber has been correctly loaded, before at least some of the one or more fluid reservoirs have been connected.

In accordance with Aspect 49, which may be used or combined with Aspect 48, the controller adjusts one or more testing values for detecting whether centrifugal separation chamber has been correctly loaded, based upon the particular fluid reservoirs of the one or more fluid reservoirs that have been connected prior to the detection.

In accordance with Aspect 50, which may be used or combined with any one of Aspects 28 through 49, the controller adjusts one or more subsequent testing values for successively detecting whether the centrifugal separation chamber has been correctly loaded, based upon any measured values associated with any previous successfully detected loadings of the centrifugal separation chambers into the centrifuge compartment.

In accordance with Aspect 51, which may be used or combined with Aspect 50, the adjustment of the one or more subsequent testing values are determined based upon the measured values from previous successfully detected loadings related to a particular biological fluid processing device.

In accordance with Aspect 52, which may be used or combined with any one of Aspects 28 through 51, the biological fluid processing device further includes an alarm coupled to the controller for generating the alert when the controller detects that the centrifugal separation chamber has not been correctly loaded into the centrifuge compartment.

In accordance with Aspect 53, which may be used or combined with any one of Aspects 28 through 52, the biological fluid processing device is a blood separation device.

In accordance with Aspect 54, a method for detecting proper loading of a centrifuge separation chamber in a biological fluid processing device is provided. The method includes receiving the centrifugal separation chamber in a centrifuge compartment of the biological fluid processing device, and spinning the received centrifugal separation chamber. A controller detects whether the centrifugal separation chamber, that has been received within the centrifuge compartment of the centrifugal separator, has been correctly loaded, and if the centrifugal separation chamber has not been loaded correctly, an alert is generated.

In accordance with Aspect 55, which may be used or combined with Aspect 54, the method further includes detecting a speed at which the centrifugal separation chamber is currently spinning, and respectively associating the detected speeds with each of one or more reference points in time.

In accordance with Aspect 56, which may be used or combined with Aspect 55, the method further includes determining a time duration for which it takes the centrifugal separator upon initiating the spinning of the received centrifugal separation chamber to reach a predetermined slow spin up testing speed, and generating the alert indicating an incorrectly loaded centrifugal separation chamber, when the time duration for which it takes the received centrifugal separation chamber to reach the predetermined slow spin up testing speed exceeds a predetermined slow spin up time duration threshold.

In accordance with Aspect 57, which may be used or combined with any one of Aspects 55 and 56, the method further includes determining that the detected speed of the received centrifugal separation chamber fails to exceed a predetermined spin up failure testing speed within a predetermined spin up failure testing time duration after initiating the spinning of the received centrifugal separation chamber, and generating the alert indicating the incorrectly loaded centrifugal separation chamber, when the detected speed of the received centrifugal separation chamber fails to exceed a predetermined spin up failure testing speed within a predetermined spin up failure testing time duration.

In accordance with Aspect 58, which may be used or combined with any one of Aspects 55 through 57, the method further includes determining a time duration for which it takes the motor of the centrifugal separator upon initiating the spinning of the received centrifugal separation chamber to reach a predetermined too fast spin up testing speed, and generating the alert indicating an incorrectly loaded centrifugal separation chamber, when the time duration for which it takes the motor to reach the predetermined too fast spin up testing speed is less than a predetermined too fast spin up time duration threshold.

In accordance with Aspect 59, which may be used or combined with any one of Aspects 55 through 58, the method further including measuring an amount of acceleration in each of multiple directions, which can be separately monitored and compared with one or more respective threshold values, and if the measured amount of acceleration in any one of the multiple directions exceeds at least one of the one or more respective threshold values, then the alert indicating an incorrectly loaded centrifugal separation chamber is produced.

In accordance with Aspect 60, which may be used or combined with Aspect 59, the amount of acceleration measured by the one or more accelerometers is analyzed by the controller after a fluid prime.

In accordance with Aspect 61, which may be used or combined with any one of Aspects 59 and 60, the amount of acceleration measured by the one or more accelerometers is analyzed by the controller after an initial spin up of the centrifugal separation chamber.

In accordance with Aspect 62, which may be used or combined with any one of Aspects 59 through 61, the amount of acceleration measured by the one or more accelerometers is continuously analyzed by the controller during the spinning of the received centrifugal separation chamber.

In accordance with Aspect 63, which may be used or combined with any one of Aspects 59 through 61, the amount of acceleration measured by the one or more accelerometers is periodically analyzed by the controller during the spinning of the received centrifugal separation chamber.

In accordance with Aspect 64, which may be used or combined with any one of Aspects 59 through 61, the amount of acceleration measured by the one or more accelerometers is analyzed by the controller at each of a predetermined set of times as determined by the timer after initiation of the spin up during the spinning of the received centrifugal separation chamber.

In accordance with Aspect 65, which may be used or combined with any one of Aspects 59 through 61, the amount of acceleration measured by the one or more accelerometers is analyzed by the controller at each of a predetermined set of speeds being reached as detected by the speed sensor after initiation of the spin up during the spinning of the received centrifugal separation chamber.

In accordance with Aspect 66, which may be used or combined with any one of Aspects 54 through 65, where a light source and light detector are communicatively coupled for producing an interface signal during a subset of the relative rotational positions of the centrifuge compartment and the received centrifugal separation chambers during which a light guide associated with the centrifugal separation chamber has a first end at least partially aligned with the light source and a second end at least partially aligned with the light detector.

In accordance with Aspect 67, which may be used or combined with Aspect 66, where a presence of the interface signal is used by the controller to detect whether the centrifugal separation chamber, which has been received within the centrifuge compartment of the centrifugal separator, has been correctly loaded.

In accordance with Aspect 68, which may be used or combined with Aspect 66, where the interface signal includes associated detectable characteristics, where the associated detectable characteristics of the interface signal are used by the controller to detect an improper loading of the centrifugal separation chamber within the centrifuge compartment of the centrifugal separator.

In accordance with Aspect 69, which may be used or combined with any one of Aspects 54 through 68, where the controller detects whether the centrifugal separation chamber, which has been received within the centrifuge compartment of the centrifugal separator, has been correctly loaded, at the end of fluid prime.

In accordance with Aspect 70, which may be used or combined with any one of Aspects 54 through 69, where the controller detects whether the centrifugal separation chamber has been correctly loaded, after a centrifuge door of the centrifuge compartment has been detected as having been opened, and subsequently closed.

In accordance with Aspect 71, which may be used or combined with any one of Aspects 54 through 70, where the controller detects whether the centrifugal separation chamber has been correctly loaded, before at least some of one or more fluid reservoirs associated with the biological fluid processing have been connected.

In accordance with Aspect 72, which may be used or combined with Aspect 71, where the controller adjusts one or more testing values for detecting whether centrifugal separation chamber has been correctly loaded, based upon the particular fluid reservoirs of the one or more fluid reservoirs that have been connected prior to the detection.

In accordance with Aspect 73, which may be used or combined with any one of Aspects 54 through 72, where the controller adjusts one or more subsequent testing values for successively detecting whether the centrifugal separation chamber has been correctly loaded, based upon any measured values associated with any previous successfully detected loadings of the centrifugal separation chambers into the centrifuge compartment.

In accordance with Aspect 74, which may be used or combined with Aspect 73, where the adjustment of the one or more subsequent testing values are determined based upon the measured values from previous successfully detected loadings related to a particular biological fluid processing device.

In accordance with Aspect 75, which may be used or combined with any one of Aspects 54 through 74, where the biological fluid processing device is a blood separation device.

It will be understood that the embodiments described above are illustrative of some of the applications of the principles of the present subject matter. Numerous modifications may be made by those skilled in the art without departing from the spirit and scope of the claimed subject matter, including those combinations of features that are individually disclosed or claimed herein. For these reasons, the scope hereof is not limited to the above description but is as set forth in the following claims, and it is understood that claims may be directed to the features hereof, including as combinations of features that are individually disclosed or claimed herein.

## Claims

1. A biological fluid processing device comprising:
a disposable flow circuit including a centrifugal separation chamber;
a centrifugal separator having a centrifuge compartment for receiving the centrifugal separation chamber of the disposable flow circuit, wherein the centrifugal separator is adapted for spinning the received centrifugal separation chamber; and
a controller configured for detecting whether any centrifugal separation chamber, that has been received within the centrifuge compartment of the centrifugal separator, has been correctly loaded, and generating an alert if the centrifugal separation chamber has not been correctly loaded.

2. The biological fluid processing device of claim 1, wherein the centrifugal separator further has a motor coupled to the received centrifugal separation chamber for spinning the received centrifugal separation chamber, where the motor is associated with a rotational speed sensor coupled to the controller for detecting a speed at which the centrifugal separation chamber is currently spinning, and a timer for respectively associating the detected speeds with each of one or more reference points in time, and wherein the controller determines a time duration for which it takes the motor of the centrifugal separator upon initiating the spinning of the received centrifugal separation chamber to reach a predetermined slow spin up testing speed, and wherein the alert indicating an incorrectly loaded centrifugal separation chamber is produced when the time duration for which it takes the motor to reach the predetermined slow spin up testing speed exceeds a predetermined slow spin up time duration threshold.

3. The biological fluid processing device of claim 2, wherein when the controller determines that the speed detected by the speed sensor fails to exceed a predetermined spin up failure testing speed within a predetermined spin up failure testing time duration after initiating the spinning of the received centrifugal separation chamber, the alert indicating the incorrectly loaded centrifugal separation chamber is produced.

4. The biological fluid processing device of any one of claims 2 through 3, wherein the controller determines a time duration for which it takes the motor of the centrifugal separator upon initiating the spinning of the received centrifugal separation chamber to reach a predetermined too fast spin up testing speed, and wherein the alert indicating an incorrectly loaded centrifugal separation chamber is produced when the time duration for which it takes the motor to reach the predetermined too fast spin up testing speed is less than a predetermined too fast spin up time duration threshold.

5. The biological fluid processing device of any one of claims 2 through 4, wherein the controller is communicatively coupled to one or more accelerometers associated with any detectable acceleration of at least one of the centrifuge compartment and the centrifugal separation chamber during the spinning by the motor.

6. The biological fluid processing device of claim 5, wherein the one or more accelerometers measure an amount of acceleration in each of multiple directions, which can be separately monitored and compared with one or more respective threshold values, and wherein if a measured amount of acceleration in any one of the multiple directions exceeds at least one of the one or more respective threshold values, then the alert indicating an incorrectly loaded centrifugal separation chamber is produced.

7. The biological fluid processing device of any one of claims 1 through 6, further comprising a light source and light detector, which are communicatively coupled for producing an interface signal during a subset of relative rotational positions of the centrifuge compartment and the received centrifugal separation chambers during which a light guide associated with the centrifugal separation chamber has a first end at least partially aligned with the light source and a second end at least partially aligned with the light detector, and wherein a presence of the interface signal is used by the controller to detect whether the centrifugal separation chamber, which has been received within the centrifuge compartment of the centrifugal separator, has been correctly loaded or wherein the interface signal includes associated detectable characteristics, wherein the associated detectable characteristics of the interface signal are used by the controller to detect an improper loading of the centrifugal separation chamber within the centrifuge compartment of the centrifugal separator.

8. The biological fluid processing device of any one of claims 1 through 7, wherein the controller adjusts one or more subsequent testing values for successively detecting whether the centrifugal separation chamber has been correctly loaded, based upon any measured values associated with any previous successfully detected loadings of the centrifugal separation chambers into the centrifuge compartment.

9. A method for detecting proper loading of a centrifuge separation chamber in a biological fluid processing device comprising:
receiving the centrifugal separation chamber in a centrifuge compartment of the biological fluid processing device;
spinning the received centrifugal separation chamber;
detecting by a controller whether the centrifugal separation chamber, that has been received within the centrifuge compartment of the centrifugal separator, has been correctly loaded; and
generating an alert if the centrifugal separation chamber has not been loaded correctly.

10. The method of claim 9, further comprising:
detecting a speed at which the centrifugal separation chamber is currently spinning;
respectively associating the detected speeds with each of one or more reference points in time;
determining a time duration for which it takes the centrifugal separator upon initiating the spinning of the received centrifugal separation chamber to reach a predetermined slow spin up testing speed; and
generating the alert indicating an incorrectly loaded centrifugal separation chamber, when the time duration for which it takes the received centrifugal separation chamber to reach the predetermined slow spin up testing speed exceeds a predetermined slow spin up time duration threshold.

11. The method of claim 10, further comprising:
determining that the detected speed of the received centrifugal separation chamber fails to exceed a predetermined spin up failure testing speed within a predetermined spin up failure testing time duration after initiating the spinning of the received centrifugal separation chamber; and
generating the alert indicating the incorrectly loaded centrifugal separation chamber, when the detected speed of the received centrifugal separation chamber fails to exceed a predetermined spin up failure testing speed within a predetermined spin up failure testing time duration.

12. The method of any one of claims 10 through 11, further comprising:
determining a time duration for which it takes a motor of the centrifugal separator upon initiating the spinning of the received centrifugal separation chamber to reach a predetermined too fast spin up testing speed; and
generating the alert indicating an incorrectly loaded centrifugal separation chamber, when the time duration for which it takes the motor to reach the predetermined too fast spin up testing speed is less than a predetermined too fast spin up time duration threshold.

13. The method of any one of claims 10 through 12, further comprising:
measuring an amount of acceleration in each of multiple directions, which can be separately monitored and compared with one or more respective threshold values; and
wherein if a measured amount of acceleration in any one of the multiple directions exceeds at least one of the one or more respective threshold values, then the alert indicating an incorrectly loaded centrifugal separation chamber is produced.

14. The method of any one of claims 9 through 13, wherein a light source and light detector are communicatively coupled for producing an interface signal during a subset of the relative rotational positions of the centrifuge compartment and the received centrifugal separation chambers during which a light guide associated with the centrifugal separation chamber has a first end at least partially aligned with the light source and a second end at least partially aligned with the light detector, wherein a presence of the interface signal is used by the controller to detect whether the centrifugal separation chamber, which has been received within the centrifuge compartment of the centrifugal separator, has been correctly loaded or wherein the interface signal includes associated detectable characteristics, wherein the associated detectable characteristics of the interface signal are used by the controller to detect an improper loading of the centrifugal separation chamber within the centrifuge compartment of the centrifugal separator.

15. The method of any one of claims 9 through 14, wherein the controller adjusts one or more subsequent testing values for successively detecting whether the centrifugal separation chamber has been correctly loaded, based upon any measured values associated with any previous successfully detected loadings of the centrifugal separation chambers into the centrifuge compartment.
